# EUROPEAN PATENT APPLICATION

(11) **EP 1 690 928 A1**
(43) Date of publication of application: **16.08.2006**
(21) Application number: 06003033.5
(22) Date of filing: 15.02.2006
(51) Int. Cl.: C12N 5/06

(54) **Laminated cell culture containing hepatocytes and method for detecting endocrine-like substance**

(30) Priority: 15.02.2005 JP 2005037037; 15.02.2005 JP 2005037038
(71) Applicant: FUJI PHOTO FILM CO., LTD., Minami-Ashigara-shi, Kanagawa 250-0193 (JP)
(72) Inventor: Aikawa, Kazuhiro c/oFuji Photo Film Co., Ltd., Minami-ashigara-shi Kanagawa 250-0193 (JP); Toda, Satoru c/oFuji Photo Film Co., Ltd., Minami-ashigara-shi Kanagawa 250-0193 (JP); Fujita, Masaharu c/oFuji Photo Film Co., Ltd., Minami-ashigara-shi Kanagawa 250-0193 (JP)
(74) Representative: HOFFMANN EITLE

(57) **Abstract**

A laminated cell culture obtained by lamination of a cell layer containing hepatocytes and a cell layer containing one or more kinds of cells selected from the group consisting of vascular endothelial cells, vascular smooth muscle cells, adipocytes, hemocytes, and macrophages; a laminated cell culture system obtained by lamination of a laminated cell culture obtainable by lamination of a cell layer containing hepatocytes and a cell layer containing one or more kinds of cells selected from the group consisting of vascular endothelial cells, vascular smooth muscle cells, adipocytes, hemocytes, and macrophages, and a cell layer containing cells for detection of an endocrine-like substance; and a method for detecting an endocrine-like substance using the laminated cell culture system.

## Description

### Technical Field

The present invention relates to a laminated cell culture, more specifically relates to a laminated cell culture obtained by lamination of a cell layer containing hepatocytes and a cell layer containing one or more kinds of cells selected from the group consisting of vascular endothelial cells, vascular smooth muscle cells, adipocytes, hemocytes, and macrophages.

The present invention also relates to a method for detecting an endocrine-like substance, more specifically relates to a method for detecting an endocrine-like substance utilizing a laminated cell culture system obtainable by lamination of a laminated cell culture obtainable by lamination of a cell layer containing hepatocytes and a cell layer containing one or more kinds of cells selected from the group consisting of vascular endothelial cells, vascular smooth muscle cells, adipocytes, hemocytes, and macrophages, and a cell layer containing cells for detecting an endocrine-like substance.

### Background Art

Presently, safety evaluation of a chemical substance is generally carried out by performing an acute toxicity test, subacute toxicity test, and chronic toxicity test by using rats or large-sized animals such as rabbits, dogs, and apes, and then extrapolating results obtained to humans. However, these methods using animals have a common problem that they are time consuming and expensive. It is desirable that the use of animals should be minimized from a viewpoint of animal protection. Further, when a metabolic product of a chemical substance in the liver is evaluated, a metabolic pattern in the liver of an animal differs from that of human, and therefore another problem is pointed out for the evaluation based on the extrapolation of animal data to humans. Accordingly, as an alternative evaluation method, establishment of an in vitro evaluation method is desired which enables evaluation of a large number of compounds in a short period of time and can be expected to contribute to researches on the difference in metabolic patterns among animal species.

As attempts to establish an in vitro evaluation method, researches of toxicity and metabolic pattern and the like have been conducted by using hepatocytes separated from animals and cultured on a petri dish as a monolayer culture, and for example, a method of using Matrigel (Bucher, N.L.R., et al., Mol. Biol. Cell, 5:312a, 1994), three-dimensional culture using collagen (Biotechnol. Appl. Biochem., 21, 19-17, 1995), a culture method using a hollow fiber (TESTLIVER-Rat-TOYOBO), and a culture method using insert cells have been reported so far. Moreover, in some researches utilizing these methods, possible usefulness of researches, in which primarily separated hepatocytes are used, not cells of an established cell line, has been demonstrated for search of metabolism patterns in animal species. However, for hepatocytes simply cultured as a monolayer on a petri dish, it is difficult to achieve long-term survival of the cells while maintaining functions thereof. Therefore, it is impossible to obtain reproducible data or evaluate long-term exposure to a chemical substance, and the aforementioned in vitro evaluation methods have their limits.

It has been revealed recently that, among medicaments, agricultural chemicals, chemical substances for industrial use and the like, some substances having a hormone-like action exist, and influences in the environment and endocrinal disturbances in living bodies caused thereby have been concerned. As for the substances generally known as environmental hormones and referred to as endocrine-like substances, intensive investigations and lots of reports have become being made (Reports of Task Force on Chemical Substance International Regulation etc., 2000 (Study on Chemicals which Affect the Endocrine Systems", March 2001, Ed. by Japan Chemical Industry Ecology-Toxicology & Information Center).

Various methods for detecting the endocrine-like substances have been studied so far. Among them, the uterus hypertrophy test and Hershberger test (Reel, J.R., et al., Fundam. Appl. Toxicol., 34:288-305), the 28-days continuous administration test (OECD Protocol for Investigating the Efficacy of the Enhanced TG407 Test Guideline (Phase 2), Rationale for the Investigation, and Description of the Protocol (Proposed on February 23, 2000)), the intrauterine exposure test (Zhou, X., et al., Nature, 361:543-547), the single generation reproduction study (Chahoud I et al., J. Comp. Neurol., 265:65-95), the two-generation reproduction toxicity study (Turner, T.T. et al., Gamete Res., 4:283-295), the multi-generation reproduction toxicity study (Carlsen, E., et al., Br. Med. J., 305 609-613) and the like are known as methods utilizing animals. As in vitro methods not utilizing animals, the cell free system receptor binding test (Howdeshell, K.L. et al., Nature, 401:763-764), the estrogen receptor binding test (Perrin, F., et al., J. Phys. Radium., 7:390-401), the reporter gene expression test using yeast (Metzger, D. et al., Nature, 334:31-34), the two-hybrid test using yeast (Mangelsdorf, D.J. et al., Cell, 83:835-839), the cell proliferation test using MCF-7 cells (Levenson, A.S. et al., Cancer Res., 57:3071-3078), the human-derived hormone receptor-induced cell line/reporter gene assay system (Mangelsdorf, D.J. et al., Cell. 83:835-839) and the like are known.

Although the aforementioned in vitro methods are more desirable than the methods using animals from the viewpoints of time consumption and cost, they cannot be applied to determination where a metabolic product is an endocrine-like substance. An in vitro method utilizing the rat S-9, which is used in mutagenicity tests, has also been attempted to solve the aforementioned problem (Inoue, T., Research Method of Bioassay for Endocrine Disturbing Chemicals, Shpringer-Verlag Tokyo, Inc.). However, desired results are not obtained, and the reason is presumed that a small amount of metabolic product produced by the metabolism of a substance over a long period of time may have an endocrine-like action (Chun, T.Y., et al., Proc. Natl. Acad. Sci. USA, 95:2325-2330). Thus, any method that enables in vitro evaluation of a metabolic product produced by metabolism over a long period of time is not known at present.

### Disclosure of the Invention

An object of the present invention is to provide a cell culture that can allow hepatocytes, isolated from an animal and cultured, to survive for a long period of time. Another object of the present invention is to provide a method that enables evaluation of toxicity of a chemical substance over long-term exposure by using said cell culture, and thereby provide a method that can minimize safety evaluation using animals and enables highly probable prediction of toxicity expression of a chemical substance to human by using primary hepatocytes of an animal or a human as hepatocytes.

A further object of the present invention is to provide a method for detecting an endocrine-like substance, more specifically, a method that can detect an endocrine-like substance that is produced during metabolism over a long period of time.

The inventors of the present invention conducted various researches to achieve the aforementioned objects, and found that a laminated cell culture of a cell layer containing hepatocytes and a cell layer containing vascular smooth muscle cells or the like can maintain metabolic abilities of hepatocytes for a long period of time about 5 to 10 times longer than that of primary hepatocytes cultured as monolayer culture. Further, the inventors of the present invention also found that an endocrine-like substance produced in metabolism over a long period of time was successfully detected by using a cell culture system comprising a cell population for metabolism activation and a cell population containing cells for detection of endocrine-like substances. The present invention was achieved on the basis of these findings.

The present invention thus provides a laminated cell culture obtained by lamination of two or more cell layers, wherein at least one of the two or more cell layers is a cell layer containing hepatocytes. According to preferred embodiments of the present invention, there are provided the aforementioned laminated cell culture, wherein at least one of the other layers of the two or more cell layers is a cell layer containing one or more kinds of cells selected from the group consisting of vascular endothelial cells, vascular smooth muscle cells, adipocytes, hemocytes, and macrophages; and the aforementioned laminated cell culture, which is obtained by adjacently laminating a cell layer containing hepatocytes and a cell layer containing one or more kinds of cells selected from the group consisting of vascular endothelial cells, vascular smooth muscle cells, adipocytes, hemocytes, and macrophages.

According to further preferred embodiments of the present invention, there are provided the aforementioned laminated cell culture according to any one of the aforementioned embodiments, wherein at least one of the two or more cell layers is a cultured cell layer which is cultured by using a carrier for a cell culture having a multi-layer structure adjacently comprising a water-containing polymer gel layer and a gel layer containing an anionic polysaccharide and a polyvalent metal ion; and the aforementioned laminated cell culture, wherein the lamination of the cell layers comprises the step of solubilization of the cell culture which contains the carrier for cell culture having a multi-layer structure adjacently comprising a water-containing polymer gel layer and a gel layer containing an anionic polysaccharide and a polyvalent metal ion.

The present invention further provides a method for safety evaluation of a substance, which uses the aforementioned laminated cell culture according to any one of the aforementioned embodiments; a method for safety evaluation of a substance, which comprises (1) the step of adding the substance to a medium containing the aforementioned laminated cell culture according to any one of the aforementioned embodiments, and (2) the step of analyzing a product in the medium after the addition of the substance; and a kit for safety evaluation of a substance, which comprises the aforementioned laminated cell culture according to any one of the aforementioned embodiments.

From another aspect, the present invention provides a cell culture system comprising a cell population for metabolism activation and a cell population containing cells for detection of an endocrine-like substance. According to preferred embodiments of the present invention, there are provided the aforementioned laminated cell culture system obtainable by lamination of a laminated cell culture obtainable by lamination of two or more cell layers and a cell layer containing cells for detection of an endocrine-like substance; the aforementioned laminated cell culture system, wherein at least one of the two or more cell layers is a cell layer containing hepatocytes, and at least one of the other two or more cell layers is a cell layer containing one or more kinds of cells selected from the group consisting of vascular endothelial cells, vascular smooth muscle cells, adipocytes, hemocytes, and macrophages; and the aforementioned laminated cell culture system, wherein the lamination of the two or more cell layers is adjacently laminating a cell layer containing hepatocytes and a cell layer containing one or more kinds of cells selected from the group consisting of vascular endothelial cells, vascular smooth muscle cells, adipocytes, hemocytes, and macrophages.

According to more preferred embodiments of the present invention, there are provided the aforementioned laminated cell culture system, wherein any one or more of the two or more cell layers and the cell layer containing cells for detection of an endocrine-like substance are cultured cell layers which are cultured by using a carrier for cell culture having a multi-layer structure adjacently comprising a water-containing polymer gel layer and a gel layer containing an anionic polysaccharide and a polyvalent metal ion; and the aforementioned laminated cell culture system, wherein at least one step of the lamination of the cell layers comprises solubilization of the cell culture which contains the carrier for cell culture having a multi-layer structure adjacently comprising a water-containing polymer gel layer and a gel layer containing an anionic polysaccharide and a polyvalent metal ion.

The present invention further provides a method for detecting an endocrine-like substance, which uses the aforementioned cell culture system according to any one of the aforementioned embodiments; a method for detecting an endocrine-like substance, which comprises (1) the step of adding a test substance to a medium containing the aforementioned laminated cell culture system according to any one of the aforementioned embodiments, and (2) the step of analyzing an endocrine-like action produced in the laminated cell culture system after the addition of the test substance; and a kit for detecting a endocrine-like substance, which comprises the aforementioned laminated cell culture system according to any one of the aforementioned embodiments.

According to the present invention, a laminated cell culture which can maintain the functions of hepatocytes for a long period of time is provided. A method for safety evaluation of a substance using this laminated cell culture is useful for determination of metabolism and toxicity of a substance such as medicaments over a long period of time.

According to the present invention, a method for detecting an endocrine-like substance which can detect an endocrine-like substance produced during metabolism over a long period of time is also provided.

### Brief Explanation of the Drawings

Fig. 1 depicts each P-450 activity of hepatocytes in a laminated cell culture according to the present invention and those cultured as monolayer culture. The P-450 (1A) activity is shown in the graph as relative values based on values immediately after inoculation, which are taken as 100.
Fig. 2 depicts each increase in AST caused by chlorpromazine in hepatocytes in a laminated cell culture according to the present invention and those cultured as monolayer culture.
Fig. 3 depicts each number of MCF-7 cells obtained in a culture with no hepatocytes (only an MCF-7 cell layer), in a culture comprising an MCF-7 cell layer laminated on a monolayer culture of rat primary hepatocytes on an insert cell, and in a laminated cell culture system according to the present invention.

### Best Mode for Carrying out the Invention

In the specification, the laminated cell culture means a culture obtainable by lamination of two or more cell layers, and includes a culture in which two or more cell layers form substantially one cell layer after they are made into a laminated cell culture. In the present invention, each of a cell layer to be laminated and a laminated cell culture is preferably in the form of a sheet.

The laminated cell culture of the present invention comprises a cell layer containing hepatocytes as the aforementioned cell layer. Each number of the layers comprising hepatocytes and that of the layers other than the cell layers comprising hepatocytes may be one or more, and each number may preferably one. The cell layer containing hepatocytes and the layer other than the cell layer containing hepatocytes are preferably laminated so that they are adjacent to each other. When they are adjacently laminated, a layer not containing cells may exist between both cell layers. Examples of the layer not containing cells include a water-containing polymer gel layer, a cell adhesive gel layer and the like which will be described later, and the cell adhesive gel layer is preferred. It is more preferred that the layer not containing cells does not exist between the two cell layers. Further, although a gap may exist between the two cell layers, the area of the gap is preferably 10% or less of the areas of the cell layers.

As the hepatocytes, any cells may be used so long as the cells are those of a tissue constituting the liver. As the cell layer other than the cell layer containing hepatocytes, a layer of cells derived from an animal is preferred, and specific examples include a cell layer containing one or more kinds of cells selected from the group consisting of vascular endothelial cells, vascular smooth muscle cells, adipocytes, hemocytes, and macrophages (including Kupffer cells). Among the cells of the aforementioned group, preferred are vascular endothelial cells and vascular smooth muscle cells. When cells which are isolated from an animal and cultured are used as the cells in the present invention, the cells may be primary culture cells or subcultured cells of an established cell line. As the hepatocytes, primarily isolated hepatocytes are preferably used. The hepatocytes and cells derived from an animal may be cells derived from any kind of mammal. Cells derived from human, bovine, dog, cat, swine, miniature pig, rabbit, hamster, rat, or mouse are preferred, and cells derived from human, rat, mouse, or bovine are more preferred. The cells contained in each of the cell layers to be laminated may be cells derived from a homologous organism or from heterogenous organisms.

A method for preparation of each of the cell layers before the lamination is not particularly limited. A commercially available cell layer may also be used as the cell layer. Preferably, as each of the cell layers, a cultured cell layer is used which is cultured by using any kind of various carriers for cell culture such as a petri dish and microplate. Further, it is preferred that cell layers, other than a cell layer serving as a substrate in the lamination, are formed on a cell carrier from which cell layer can be removed.

As the carrier for cell culture, any of known carriers for cell culture may be used, including the carriers for cell culture described below from which a cell layer can be removed.

Examples of the carrier for cell culture from which cell layer can be removed include, but not particularly limited thereto, the carriers for cell culture described in Japanese Patent No. 3261456, Japanese Patent Publication (KOKOKU) No. 6-104061 and the like. Particularly preferred examples include a carrier for cell culture having a multi-layer structure adjacently comprising a water-containing polymer gel layer and a gel layer containing an anionic polysaccharide and a polyvalent metal ion (hereinafter also referred to as "the subject carrier for cell culture").

The water-containing polymer gel means a hydrophilic polymer, and in particular, means a water absorbing polymer which is insoluble in water but maintains water in the polymer to have a two- or three-dimensional support structure throughout the system. In the subject carrier for cell culture, as a water-containing polymer gel layer, a layer is used which allows a substance such as a chelating agent to move from one surface of the layer and reach the other surface by diffusion in the layer. Further, in the subject carrier for cell culture, as the water-containing polymer gel layer, a layer is used which does not allow a gel containing an anionic polysaccharide and a polyvalent metal ion such as alginic acid gel to move from one surface of the layer and reach the other surface. The water-containing polymer gel layer used in the preparation of the subject carrier for cell culture is not particularly limited so far that the layer is such a layer as those mentioned above, and may consist of a synthetic polymer, natural polymer, or biopolymer. Examples of the water-containing polymer gel include acrylamide gel, crosslinked acrylic acid gel, agarose, gelatin, dextran, chitosan, silica gel, and the like. Chitosan is preferably used.

In the subject carrier for cell culture, the "water-containing polymer gel layer" is preferably a support. The support in a carrier for cell culture means herein a layer that serves as a substrate in the preparation of a carrier for cell culture having a multi-layer structure.

In the subject carrier for cell culture, the water-containing polymer gel layer preferably has a thickness of 0.01 *µ*m or more and 5 *µ*m or less, more preferably 0.1 µm or more and 4 *µ*m or less, still more preferably 0.5 *µ*m or more and 3 *µ*m or less. In the specification, the thickness of a layer means a thickness measured for the layer in a sufficiently dried state, unless otherwise indicated. In the specification, such a thickness of a layer is also referred to as "dry membrane thickness". The thickness of a layer can be measured by using a section image of electron microscope, membrane thickness micrometer, ellipsometer, angle adjustable XPS, optical interferometric membrane thickness meter and the like. The thickness can be preferably measured by using a membrane thickness micrometer, section image of electron microscope, or an optical interferometric membrane thickness meter.

The water-containing polymer gel layer of the subject carrier for cell culture can be prepared by various generally known methods for preparation of a water-containing polymer gel membrane. Examples of the methods include a method of casting a solution of a water-containing polymer gel (casting method), a method of coating such a solution by using a bar coater (bar coating method), a method of coating such a solution by using a gap coater (gap coating method), and the like. Among them, the bar coating method and the gap coating method are preferred.

Examples of the anionic polysaccharide in the subject carrier for cell culture include alginic acid, dextran sulfate, carboxymethylcellulose, carboxymethyldextran, hyaluronic acid, and the like. Alginic acid is preferably used.

Alginic acid exists in nature as a cell wall-constituting polysaccharide or intercellular filling substance of brown algae, and can be obtained from the algae as raw materials. Specific examples of the brown algae as a raw material include brown algae belonging to Order Fucales, Family Durvilleaceae, Genus Durvillea (e.g., D. potatorum), Order Fucales, Family Fucaceae, Genus Ascophyllum (e.g., A. nodosum), Order Laminariales, Family Laminariaceae, Genus Laminaria (e.g., Laminaria japonica, Laminaria longissima), Order Laminariales, Family Laminariaceae, Genus Eisenia (e.g., Eisenia bicyclis), Order Laminariales, Family Laminariaceae, Genus Ecklonia (e.g., Ecklonia cava, Ecklonia kurome), and Order Laminariales, Family Lessoniaceae, Genus Lessonia (e.g., L. flavikans). Commercially available alginic acid can also be used. A G/M ratio of alginic acid is not particularly limited. A larger G/M ratio provides a higher gel formation ability, and accordingly, a larger G/M ratio is more preferred. Specifically, the ratio may preferably be from 0.1 to 1, more preferably from 0.2 to 0.5 (numerical ranges indicated by "from -- to --" in the specification include lower and upper limits).

The "alginic acid gel" means alginic acid gelled by a chelate structure formed with a carboxylic acid group in the molecule of alginic acid and a polyvalent metal ion, and "alginic acid gel layer" specifically referred to in the present specification means alginic acid gel in the form of a layer. Alginic acid is a block copolymer consisting of glucuronic acid (G) and mannuronic acid (M), and it is considered that the polyvalent metal cation enters into a pocket structure of the M block to form an egg box and thereby cause the gelation. Specific examples of the polyvalent metal cation that can cause the gelation of alginic acid include, for example, metal ions such as barium (Ba), lead (Pb), copper (Cu), strontium (Sr), cadmium (Cd), calcium (Ca), zinc (Zn), nickel (Ni), cobalt (Co), manganese (Mn), iron (Fe) and magnesium (Mg) ions. Among them, particularly preferred are calcium ion, magnesium ion, barium ion and strontium ion. The "alginic acid gel" may be a polyion complex gel of alginic acid and an organic polymer compound having a cationic residue. Examples of the organic polymer compound having a cationic residue mentioned herein include compounds having two or more amino groups such as polylysine, chitosan, gelatin, and collagen.

The gelation of alginic acid may be achieved in a conventional manner. For example, the gelation of alginic acid can be carried out by using ion exchange. For example, when calcium ions are added to an aqueous solution of sodium alginate, ion exchange quickly occurs to give calcium alginate gel. More specifically, an 0.2 to 5% aqueous solution of sodium alginate may be applied on a water-containing polymer gel (for example, chitosan) layer, which is then immersed in a 0.01 to 1.0 M aqueous solution of calcium chloride for soak with calcium chloride, and then left at 20 to 30°C for 3 minutes to 3 hours to obtain an calcium alginate gel layer. If gelation of alginic acid is attained by using a water-containing polymer as mentioned above, a carrier for cell culture comprising a water-containing polymer gel layer and an alginic acid gel layer formed thereon can be obtained.

The gel layer containing an anionic polysaccharide and a polyvalent metal ion of the subject carrier for cell culture preferably has a thickness of 0.01 *µ*m or more and 50 *µ*m or less, more preferably 0.1 *µ*m or more and 10 *µ*m or less, still more preferably 0.5 *µ*m or more and 5 *µ*m or less. When the solid content in the alginic acid gel layer is too low, a problem arises that a layer consisting of a satisfactory membrane cannot be formed and holes are formed, whereas when the content is too high, different problems of curl or fracture of a dried membrane, deformation during a culture process, or insufficient solubilization in the alginic acid gel solubilization step may arise.

The subject carrier for cell culture may further have a cell adhesive gel layer on the outermost surface of the cell culture surface side.

The "cell adhesive gel layer" means a hydrogel in the form of a layer having a cell adhesion property. The layer may comprise any natural or synthetic substances so long as they do not have cytotoxicity and can form a gel to which cells adhere under an ordinary culture condition, and preferably consists of a layered extracellular matrix component gel. The extracellular matrix is generally defined as "a stable biological structure existing extracellularly in an animal tissue and a complex aggregate formed by biological polymers which are synthesized by cells, and secreted and accumulated outside the cells" (Dictionary of Biochemistry (3rd edition), p.570, Tokyo Kagaku Dojin), and the matrix plays roles of physically supporting cells, regulating cellular activities (i.e., a role of transmitting extracellular information to a cell to change its activities) and the like. The "extracellular matrix component" means a constituting ingredient of the extracellular matrix. Specific examples include collagen, elastin, proteoglycan, glucosaminoglycan (hyaluronic acid, chondroitin sulfate, dermatan sulfate, heparan sulfate, heparin, keratan sulfate and the like), fibronectin, laminin, vitronectin, gelatin and the like. Among them, especially preferred examples include collagen, atelocollagen, Matrigel (gel consisting of type IV collagen, laminin and heparan sulfate), hyaluronic acid and gelatin. The extracellular matrix component can be obtained in a conventional manner, and commercially available extracellular matrix components may also be used. The cell adhesion component can be gelled in a conventional manner. For example, when the cell adhesion component is collagen, a collagen gel can be obtained by incubating a 0.3 to 0.5% aqueous solution of collagen at 37°C for from 10 to 20 minutes. A gelling agent may be used for the gelation of the extracellular matrix component, if needed.

The cell adhesive gel layer in the subject carrier for cell culture preferably has a thickness of 0.005 *µ*m or more and 5.0 *µ*m or less, more preferably 0.005 *µ*m or more and 1.0 µ m or less, still more preferably 0.005 µ m or more and 0.5 µ m or less. If the cell adhesive gel layer is too thick, cracks may be generated on the layer during drying, and further a cell transfer may become markedly difficult.

When the cell adhesive gel layer is formed on the gel layer containing an anionic polysaccharide and a polyvalent metal ion, the gel layer containing an anionic polysaccharide and a polyvalent metal ion and the cell adhesive gel layer may be prepared separately and then laminated. It is preferred that an aqueous solution containing a cell adhesive component is added on the gel layer containing an anionic polysaccharide and a polyvalent metal ion, and then the aqueous solution is gelled. This is because the cell adhesive gel layer does not have sufficient physical strength for lamination or delamination, and therefore it is difficult to delaminate the cell adhesive gel layer from a container (for example, dish, petri dish or the like) in which the cell adhesive gel layer is formed. Further, the cell adhesive gel layer as an extremely thin layer can be conveniently obtained by immersing the gel layer containing an anionic polysaccharide and a polyvalent metal ion in a solution containing a cell adhesive component (immersion method), coating the gel layer with the solution (coating method), or casting the solution on the gel layer (casting method). Any of the above methods can be used for the preparation of the subject carrier for cell culture. Among them, the casting method is preferably used. For example, when a collagen gel layer is formed on an alginic acid gel layer, a commercially available 0.3 to 0.5% aqueous solution of collagen may be diluted to an appropriate concentration as required, and then the resulting solution may be cast on a calcium alginate gel prepared by the method described above and dried to obtain a collagen gel layer formed on alginic acid gel layer.

The cultured cell layer formed on the subject carrier for cell culture can be delaminated as a cell sheet by a solubilization treatment of the gel layer containing an anionic polysaccharide and a polyvalent metal ion. Therefore, by using a cultured cell layer formed on the subject carrier for cell culture as the cell layer containing hepatocytes or other cell layers, the laminated cell culture of the present invention can be easily manufactured.

The solubilization treatment of the gel layer containing an anionic polysaccharide and a polyvalent metal ion can be carried out by removing cation components that constitute the gel layer containing an anionic polysaccharide and a polyvalent metal ion. When the cation is a polyvalent metal ion, the treatment can be performed by subjecting the carrier for cell culture on which a cultured cell layer is formed to 1) immersion in a medium added with an ion that forms a chelate or a hardly soluble salt with the polyvalent metal ion, such as phosphate ion, 2) immersion in a medium added with an aqueous solution of a chelating agent, 3) immersion in a medium in which polyvalent metal ions are reduced, or 4) masking of the polyvalent metal ion in the culture medium of the cells with a chelating agent. A medium for cell culture generally contains a lot of phosphate ions. Therefore, the solubilization treatment of the gel layer containing an anionic polysaccharide and a polyvalent metal ion is preferably performed by using a medium having a concentration of the polyvalent metal ion lower than that of a minimum medium generally used for cell culture and further containing a chelating agent. Specifically, the concentration is preferably 2.6 mM or less, more preferably 3 *µ*M or less, still more preferably 0.5 *µ*M or less. Most preferably, the concentration is substantially zero. The concentration of the chelating agent is preferably 2.3 mM or more and 26,000 mM or less, more preferably 2.3 mM or more and 2,600 mM or less. By using such a medium in which polyvalent metal ions are reduced as mentioned above, solubilization of the gel layer containing an anionic polysaccharide and a polyvalent metal ion can be achieved with reduced invasion of the chelating agent into cells.

Examples for the chelating agent used for the solubilization treatment include, for example, ethylenediamine-di-orthohydroxyphenylacetic acid, diaminopropanetetraacetic acid, nitrilotriacetic acid, hydroxyethylethylenediaminetriacetic acid, dihydroxyethylglycine, ethylenediaminediacetic acid, ethylenediaminedipropionic acid, iminodiacetic acid, diethylenetriaminepentaacetic acid, hydroxyethyliminodiacetic acid, 1,3-diaminopropanoltetraacetic acid, triethylenetetraminehexaacetic acid, transcyclohexanediaminetetraacetic acid, ethylenediaminetetraacetic acid (EDTA), glycol ether-diaminetetraacetic acid, O,O'-bis(2-aminoethyl)ethylene glycol-N,N,N',N'-tetraacetic acid (EGTA), ethylenediaminetetrakismethylenephosphonic acid, diethylenetriaminepentamethylenephosphonic acid, nitrilotrimethylenephosphonic acid, 1-hydroxyethylidene-1,1-diphosphonic acid, 1,1-diphosphonoethane-2-carboxylic acid, 2-phosphonobutane-1,2,4-tricarboxylic acid, 1-hydroxy-1-phosphonopropane-1,3,3-tricarboxylic acid, catechol-3,5-disulfonic acid, sodium pyrophosphate, sodium tetrapolyphosphate, sodium hexametaphosphate, 1-hydroxypropylidene-1,1-diphosphonic acid, 1-aminoethylidene-1,1-diphosphonic acid and salts thereof. Among them, particularly preferred examples include EDTA, EGTA, ethylenediaminetetraphosphonic acid, and 1-hydroxyethylidene-1,1-diphosphonic acid.

In the medium for the solubilization treatment of the gel layer containing an anionic polysaccharide and a polyvalent metal ion, a concentration of cationic amino acids is preferably less than that of cationic amino acids in a minimum medium generally used for cell culture. Specifically, the concentration is preferably 1.0 mM or less, more preferably 2 *µ*M or less, still more preferably 0.5 *µ*M or less. Most preferably, the concentration is substantially zero. The cationic amino acid components means L-Lysin (Lys), L-Arginine (Arg), L-Histidine (His), L-Cystine (Cys) and salts thereof.

The solubilization treatment of the gel layer containing an anionic polysaccharide and a polyvalent metal ion may be performed by one or more times of immersions in any of the above mediums for the solubilization treatment. When two or more times of immersions are conducted, the mediums for the solubilization treatment may be the same or different.

For the solubilization treatment of the gel layer containing an anionic polysaccharide and a polyvalent metal ion, i.e., for the immersion of the carrier for cell culture in the medium for the solubilization treatment, on which a cultured cell layer is formed, the immersion is preferably performed so that the chelating agent infiltrates from the water-containing polymer gel layer side. By such an operation, the water-containing polymer gel layer and the gel layer containing an anionic polysaccharide and a polyvalent metal ion can be easily separated, and a cell sheet containing cultured cell can be easily delaminated from the water-containing polymer gel layer. It is not necessary to completely remove the gel layer containing an anionic polysaccharide and a polyvalent metal ion by the solubilization treatment of the gel layer containing an anionic polysaccharide and a polyvalent metal ion, and the gel layer containing an anionic polysaccharide and a polyvalent metal ion remained insolubilized may be left on the cell sheet. However, the gel layer containing an anionic polysaccharide and a polyvalent metal ion is preferably solubilized and removed as much as possible.

The cultured cell layer formed on the subject carrier for cell culture can be delaminated as a cell sheet by the solubilization treatment of the gel layer containing an anionic polysaccharide and a polyvalent metal ion as described above. The subject carrier for cell culture may be provided with a physical reinforcing means on the surface of the water-containing polymer gel layer on the side opposite to the cell adhesive gel layer to improve operability of the carrier at the time of the solubilization treatment. A material of the physical reinforcing means is not particularly limited so long as the material does not affect cells. Examples include metals (for example, iron, stainless steel, titanium, gold, and the like), plastics (for example, polystyrene, polycarbonate, polyethylene, polypropylene, acrylic plastics, and the like), inorganic materials such as ceramics. Preferred are stainless steel, titanium, and plastics.

The physical reinforcing means may be in any form so long as the means can improve the operability of the subject carrier for cell culture. The physical reinforcing means is preferably in the form of a plate, and preferably has a thickness of 0.1 *µ*m or more and 10 mm or less, more preferably 1 *µ*m or more and 1 mm or less, still more preferably 10 *µ*m or more and 200 *µ*m or less.

The physical reinforcing means may be in any shape so long as the means has a part through which the subject carrier for cell culture is visible for observation of cells. Examples of the shape include a circle, polygon (triangle, quadrilateral, hexagon and the like), combinations thereof (sector and the like) and the like. Among them, a circle-like shape is preferred. One or more of the parts through which the subject carrier for cell culture is visible may be provided. Further, the physical reinforcing means preferably has an asymmetric shape so that the side of the water-containing polymer gel layer adhered with the physical reinforcing means can be easily identified.

The physical reinforcing means may be adhered to the water-containing polymer gel membrane by any method so long as cell culture is not affected. For example, adhesion may be attained by a method of using a commercially available adhesive (for example, Aronalpha, Bond and the like) after preparation of the polymer gel membrane, or putting the physical reinforcing means on an undried water-containing polymer gel membrane.

Although it depends on a type of the material, the physical reinforcing means may sometimes have a sharp edge. When such means is used, it is concerned that the physical reinforcing means may break the water-containing polymer gel with the sharp edge thereof, or may harm an operator handling the means. Therefore, it is preferable to eliminate such sharp edge. The sharp edge can be removed by any method so long as the method does not influence the cell culture. Examples of the method include physical polishing (for example, polishing by using a file or the like) and chemical treatments (for example, chemical etching or the like). For the subject carrier for cell culture, when the physical reinforcing means is made of stainless steel, the means is preferably subjected to a chemical treatment such as chemical etching.

For the subject carrier for cell culture having the cell adhesive gel layer on the outermost surface of the cell culture side, a part not modified with the cell adhesive gel layer, i.e., a part wherein the cell adhesive gel layer is not formed, may be provided on a part of the outermost surface for cell culture to improve operability for the delamination of a cultured cell layer formed on the subject carrier for cell culture as a cell sheet. The part not modified with the cell adhesive gel layer thus consists of the water-containing polymer gel layer or the gel layer containing an anionic polysaccharide and a polyvalent metal ion. By providing the part not modified with the cell adhesive gel layer, the handling property for the delamination of the water-containing polymer gel layer from the cell adhesive gel layer can be improved. That is, by pinching the water-containing polymer gel layer of the part not modified with the cell adhesive gel layer with a pair of tweezers or the like, the water-containing polymer gel layer can be removed without touch to the cell adhesive gel layer, and therefore the cells are less adversely affected. The part not modified with the cell adhesive gel layer is preferably provided at a corner of the water-containing polymer gel layer of the subject carrier for cell culture.

As a method for providing the part not modified with the cell adhesive gel layer, any method can be used so long as the method does not disturb the cell culture. Examples include the generally well-known masking method, that is, a method of covering beforehand a part on a water-containing polymer gel layer to be not modified with the cell adhesive gel layer with another material, then modifying the water-containing polymer gel layer with a cell adhesive gel component, and removing the material covering the part to obtain the part not modified with the cell adhesive gel layer.

A material as the aforementioned other material used for the coverage to provide the part not modified with the cell adhesive gel layer is not limited so long as the material causes no problem in cell culture. Examples include silicon rubber, commercially available masking tapes or adhesive tapes, plastics (for example, polystyrene, polycarbonate, polyethylene, polypropylene, acrylic plastics, and the like), metals (for example, iron, stainless steel, titanium, gold, and the like), and silicon rubber and commercially available masking tapes are preferred.

A shape of the part not modified with the cell adhesive gel layer is not particularly limited so long as the cell culture is not disturbed. A circle, polygon (triangle, quadrilateral, hexagon, or the like), or combination thereof (sector or the like) is preferred. Triangle or sector is more preferred. An area of the part not modified with the cell adhesive gel layer is not particularly limited so long as cell culture is not disturbed. The part preferably has a diameter of 0.1 mm or more and 10 mm or less, more preferably diameter of 1 mm or more and 5 mm or less, as a circle.

When the subject carrier for cell culture is prepared, a preparation solution containing a carbodiimide may be used to improve adhesiveness. Carbodiimide and N-hydroxysuccinimide may be added to a preparation solution for any layer. Preferable examples include addition to a preparation solution for the gel layer containing an anionic polysaccharide and a polyvalent metal ion, or preliminary soaking in the water-containing polymer gel, or alternatively, immersion in a solution in which calcium chloride is co-dissolved therewith after the application of the gel layer containing an anionic polysaccharide and a polyvalent metal ion. The carbodiimide is preferably a water-soluble carbodiimide. Examples include 1-ethyl-3-(3-dimethylaminopropyl)carbodiimide hydrochloride. When the carbodiimide is used, a concentration of 0.01 mg/l or more and 200 g/l or less may be preferred. In the above process, N-hydroxysuccinimide may be used as a catalyst, and a concentration thereof is preferably 1 mass % or more and 50 mass % or less based on the carbodiimide.

In the subject carrier for cell culture, it is preferable not to use carbodiimide unless particularly needed.

The subject carrier for cell culture may be sterilized by any method. Sterilization by radiation such as electron beam, γ -ray, X-ray and ultraviolet ray is preferably used. An electron beam, γ -ray and ultraviolet ray are more preferably used, and electron beam sterilization is particularly preferred. Exposure dose for the electron beam sterilization is preferably 0.1 kGy or more and 65 kGy or less, particularly preferably 1 kGy or more and 40 kGy or less. Chemical sterilization such as ethylene oxide gas (EOG) sterilization and sterilization using a high temperature such as high pressure steam gas sterilization are not preferred, because the cell adhesive layer and the gel layer containing an anionic polysaccharide and a polyvalent metal ion may be decomposed. The carrier for cell culture sterilized as described above can be stored at room temperature for a long period of time, if it is stored under a sterile condition.

The aforementioned sterilization methods may be used individually or in combination. The same sterilization method may be applied repeatedly.

When hepatocytes or cells contained in the other layers, such as vascular endothelial cells, vascular smooth muscle cells, adipocytes, hemocytes, and macrophages, are cultured by using the subject carrier for cell culture, a culture medium (for example, D-MEM medium, MEM medium, HamF12 medium, or HamF10 medium) containing cells at a density of from 10,000 to 15,000 cells/ml is usually added onto the surface of the carrier for cell culture (when the carrier has a cell adhesive gel layer, added onto the cell adhesive gel layer). The cell culture conditions can be appropriately chosen depending on the type of cells to be cultured. When the cells are cultured on the cell adhesive gel layer, in general, the culture is preferably continued until a confluent cell monolayer is formed on the cell adhesive gel layer.

Culture for preparing a cell layer can be performed, for example, as follows. The carrier for cell culture is placed inside a petri dish or the like, then an appropriate culture medium (for example, D-MEM medium, MEM medium, HamF12 medium, HamF10 medium) is added to the petri dish to immerse the carrier for 5 minutes, and then the medium is exchanged. After this procedure is repeated three times, the culture system was left for 12 to 24 hours so that the culture medium can infiltrate into the carrier for cell culture. Then, the culture medium in the petri dish is discarded, and then cells are inoculated onto the cell adhesive gel layer of the carrier for cell culture, and further an appropriate culture medium (for example, D-MEM medium, MEM medium, HamF12 medium, HamF10 medium) is added to the petri dish. After the system is left at 37°C for 1 to 2 hours so that the cells can be held by (adhered to) the cell adhesive gel layer, the culture is continued at 37°C. During the culture, the culture medium may be exchanged, if needed. Usually, the culture medium is exchanged every 0.5 to 2 days of the culture.

Examples of the method for lamination of the cell layers used in the present invention include a method of using a carrier for cell culture, a commercially available collagen membrane or the like, and the method is not particularly limited.

When a carrier for cell culture having a multi-layer structure adjacently comprising a water-containing polymer gel layer and a gel layer containing an anionic polysaccharide and a polyvalent metal ion is used, the lamination of cell layers can be performed by superimposing a cell culture cultured by using the subject carrier for cell culture on a cell layer serving as a substrate so that the cell culture surfaces face to each other, with or without applying a weight, further culturing the cells, and then solubilizing the gel layer containing an anionic polysaccharide and a polyvalent metal ion, or by superimposing a cell sheet comprising a cell layer obtained by solubilizing a cell culture cultured by using the subject carrier for cell culture on a cell layer serving as a substrate, and similarly culturing the cells.

The method for cell culture with applying a weight may be any method so long as sufficient weight is applied so that unevenness should not be formed in cells or substrate on which cells are transferred. If cells are sealed by weighting, cells may be smothered. Therefore, also for the cell layer serving as a substrate, at least either of the cell culture substrates is preferably held on a carrier for cell culture comprising a water-permeable gel, porous membrane, or a combination thereof. Further, for uniform transfer, a weight should be applied so as to sufficiently cover the surface of the cell layer. However, uniform contact may disturb diffusion of oxygen, and therefore, a weight may preferably be applied through non-woven fabric (nylon, polyester, stainless steel and the like) or the like so as not to disturb diffusion of oxygen.

In the cell culture method with weighting, the weight to be applied is preferably from 0.1 to 50 g/cm², more preferably from 0.5 to 10 g/cm². The culture time of the cells under weighting is not particularly limited, and can be appropriately chosen so that sufficient transfer of cells can be achieved. The period of time is preferably 4 to 72 hours, more preferably from 6 to 48 hours. In the present invention, the culture is preferably performed without weighting.

Either one of the cell layer containing hepatocytes and the other cell layer may be used as a cell layer serving as a substrate. Further, it is also possible to prepare both cell layers on carriers for cell culture having a multi-layer structure adjacently comprising a water-containing polymer gel layer and a gel layer containing an anionic polysaccharide and a polyvalent metal ion, and solubilize the gel layer containing an anionic polysaccharide and a polyvalent metal ion in both or either of the carriers for cell culture to attain the lamination.

The culture of the laminated culture of the present invention may be performed by using any of culture flask, culture tube, petri dish, microplate and insert cell as a culture vessel. It is preferable to perform the culture by using a microplate or insert cell. The culture can be performed at a temperature of 25 to 37°C and a CO₂ concentration of 0 to 6%. For the cultures, mediums including, for example, D-MEM medium, MEM medium, HamF12 medium, HamF10 medium and the like can be used.

The laminated culture of the present invention can maintain metabolic abilities of hepatocytes for a prolonged period of time compared with hepatocytes cultured as monolayer culture. Accordingly, it is possible to evaluate metabolism of a substance in hepatocytes over a long period of time by using the laminated culture of the present invention. Therefore, by using the laminated culture of the present invention, safety of a variety kind of substances can be more accurately evaluated. Although the substances are not particularly limited, examples include chemical substances such as medicaments that may be entered into the inside of human bodies. Specifically, examples include medicaments, common chemical substances, environmental pollutants, natural substances and the like.

The method for safety evaluation of a substance according to the present invention preferably comprises:
(1) the step of adding the substance to a medium containing the laminated cell cultures of the present invention, and
(2) the step of analyzing a product in the medium after the addition of the substance. For this method, mediums including, for example, D-MEM medium, MEM medium, HamF12 medium, HamF10 medium and the like can be used as the medium. Examples of the analysis include identification and quantification of a metabolic product and the like, specifically, identification and quantification of a metabolic product toxicated or detoxicated from a starting material. By analyzing a product in the medium containing the laminated cell culture of the present invention, metabolic patterns in the liver of various animals can be determined in a short period of time.

In the specification, the endocrine-like substance means a substance having an endocrine disturbance action, and includes substances having a hormone-like action, and substances inducing production of a hormone or substances having a hormone-like action.

As cells for detection of the endocrine-like substance, known cells for the detection can be used, and they are not particularly limited. For example, examples of cells for detection of estrogenic substance include MCF-7 (produced by Dainippon Pharmaceutical), T47D (produced by Dainippon Pharmaceutical), HeLa (produced by Dainippon Pharmaceutical), PC12 (produced by Dainippon Pharmaceutical) and the like. However, the cells are not limited to these examples, and any cells expressing estrogen receptors can be used. Similarly, as cells for detection of endrogen, any cells expressing endrogen receptors can be used.

The cell population means a population containing two or more homologous or heterogenous cells. A shape of the cell population is not particularly limited. The shape of a layer or a membrane is preferred, more preferably the shape may be in the form of a sheet.

The cell population for metabolism activation and the cell population containing cells for detection of an endocrine-like substance are preferably adhered to each other. The cell population for metabolism activation or the cell population containing cells for detection of an endocrine-like substance can also be cultured on insert cells and used within the same culture vessel.

The cell population for metabolism activation means a cell population that can promote metabolism or can maintain metabolic functions for a long period of time, and examples include, for example, a cell population containing two or more kinds of cells including cells having metabolic functions and cells that promote and maintain metabolism. Examples of such a cell population include, for example, a cell population containing hepatocytes and vascular endothelial cells, more specifically, a cell population in which rat primary hepatocytes and rat endothelial cell are co-cultured. As the cell population for metabolism activation, a laminated cell culture obtained by lamination of cell layers each containing a different type of cells is preferred.

According to the above invention, the laminated cell culture system means a culture obtainable by lamination of the aforementioned laminated cell culture and a cell layer containing cells for detection of an endocrine-like substance, and also includes a culture in which the cell layer of the laminated cell culture and the cell layer containing cells for detection of an endocrine-like substance form substantially one cell layer after they are made into the laminated cell culture. The cell layer containing cells for detection of an endocrine-like substance is preferably in the form of a sheet.

The methods for preparing the cell layer before the lamination of the aforementioned laminated cell culture and the cell layer containing cells for detection of an endocrine-like substance are not particularly limited, and a commercially available cell layer may be used as the cell layer. Preferably, a cultured cell layer which is cultured by using any one of various carriers for cell culture such as petri dish and microplate is used as each cell layer. Further, it is preferred that cell layers other than the cell layer that serves as a substrate at the time of the lamination are formed on cell carriers from which the cell layers can be removed.

At the time of preparation of the laminated cell culture used for the present invention or preparation of the laminated cell culture system of the present invention, any cell layer or a laminated cell culture may be used as a substrate. Further, both cell layers can be formed on a carriers for cell culture, which has a multi-layer structure adjacently comprising a water-containing polymer gel layer and a gel layer containing an anionic polysaccharide and a polyvalent metal ion, and then the gel layer containing an anionic polysaccharide and a polyvalent metal ion on both or one of the carriers for cell culture can be solubilized to attain the lamination.

In the cell culture system of the present invention, it is possible to maintain functions of cells contained in the cell culture system for a long period of time as long as about 60 days. Therefore, the method enables detection of a endocrine-like substance produced in metabolism over a long period of time and investigation of an endocrine-like action over a long period of time.

The method for detecting an endocrine-like substance of the present invention preferably comprises:
(1) the step of adding a test substance to a medium containing the laminated cell culture system, and
(2) the step of analyzing an endocrine-like action produced in the laminated cell culture system after the addition of the test substance.
Analysis of the endocrine-like action can be conducted by analysis of the cells for detection of endocrine-like substances. Examples of the analysis include, specifically, determination of number of cells for detection of endocrine-like substances, determination of galactose activity, measurement of fluorescence emitted by luciferase and the like in the laminated cell culture system in which culture is performed for about 60 days after the addition of the test substance,.

### Examples

The present invention will be explained more specifically with reference to the following examples. However, the scope of the present invention is not limited to the following examples.

### [Example 1] Preparation of carrier for cell culture

### (1) Preparation of water-containing chitosan gel membrane

500 g of 1 mass % acetic acid solution was gradually added with 6 g of Chitosan 100D (Dainichiseika Colour & Chemicals Mfg. Co., Ltd.), and stirred at room temperature for 7 hours for dissolution. The solution was filtered through a microfilter FG-30 produced by Fuji Photo Film Co., Ltd.

The filtered acetic acid solution of chitosan was applied to a polyethylene terephthalate film (length: 20 cm, width: 20 cm, film thickness: 190 µm) by using an applicator so that a dry membrane thickness of 2 µm was obtained, and dried at 37°C overnight. The resulting membrane was immersed in a 1.9 mass % solution of sodium hydroxide in methanol for 30 minutes, and subsequently in PBS (Dulbecco's phosphate-buffered saline) for 30 minutes. Then, the membrane was immersed in a distilled water bath for 30 minutes to obtain a chitosan gel membrane. The chitosan gel membrane obtained as described above was dried overnight at room temperature, and stored in a plastic bag.

### (2) Preparation of water-containing chitosan polymer gel membrane/calcium alginate laminated membrane

On the chitosan gel membrane obtained in (1) mentioned above, 2 mass % aqueous solution of Kimica Algin B-1 (Kimica Corporation) was applied by using an applicator so that a dry membrane thickness of 6 *µ*m was obtained. This application product was immersed in a 25% methanol aqueous solution bath containing 0.03 mol/l of calcium chloride for 20 minutes and then immersed in a distilled water bath for 30 minutes to obtain a water-containing chitosan gel/calcium alginate laminated gel membrane.

### (3) Modification with Collagen layer

On the water-containing chitosan polymer gel/calcium alginate laminated gel membrane obtained in (2) mentioned above and not dried, a frame made of silicone rubber and metal aluminum (internal length: 12 cm, internal width: 7 cm, thickness: 5 mm) was placed. 8 ml of Cellmatrix I-P (Nitta Gelatin Co., Ltd.) was added with 1 ml of Ham's F-12 medium concentrated 10 times and mixed by using a stirrer for 1 minute under ice cooling. Then, this mixture was further added with 1 ml of buffer (obtained by dissolving 2.2 g or 4.7 g of NaHCO₃ in 100 ml of 0.05 N aqueous NaOH) and mixed under ice cooling in such a manner that the mixture was not foamed. 6.5 ml of this solution was cast in the internal space of the aforementioned flame. The cast membrane was dried overnight at room temperature, washed with distilled water and then dried again to obtain a water-containing chitosan polymer gel/calcium alginate/collagen layer-modified laminated membrane.

### [Example 2] Preparation of laminated cell culture

Rat primary hepatocytes (collected by the method described in "Techniques of Tissue Culture, Second edition", Ed. by Japan Tissue Culture Association, Asakura Publishing, p.135) were inoculated on a 12-well microplate (FALCON, No. 3505) in an amount of 50000 cells/well and cultured for 24 hours under the conditions of 37°C and 5% CO₂. Separately, the carrier for cell culture prepared in Example 1 was adhered to bottom of a 20-mm petri dish (FALCON, No. 2163) with a small amount of sterilized water. 20,000 cells of rat vascular endothelial cells A-10 (Dainippon Pharmaceutical Co., Ltd.) were inoculated on the carrier for cell culture and cultured for 72 hours under the conditions of 37°C and 5% CO₂. As the medium, 10% FBS Eagle's MEM medium (GIBCO) was used for both cultures. The rat vascular endothelial cell culture obtained was carefully placed on the aforementioned rat primary hepatocytes on the 12-well microplate so that the cell growing side contacted with the rat primary hepatocytes, and cultured for 24 hours under the conditions of 37°C and 5% CO₂. After the culture, 1 mM ethylenediaminetetrasulfonic acid was added to the culture, the cells were cultured for 10 minutes under the conditions of 37°C and 5% CO₂, and the chitosan membrane coming up to the upper surface was removed by using a pair of tweezers to obtain a laminated cell culture.

### [Example 3] P-450 activity in laminated cell culture

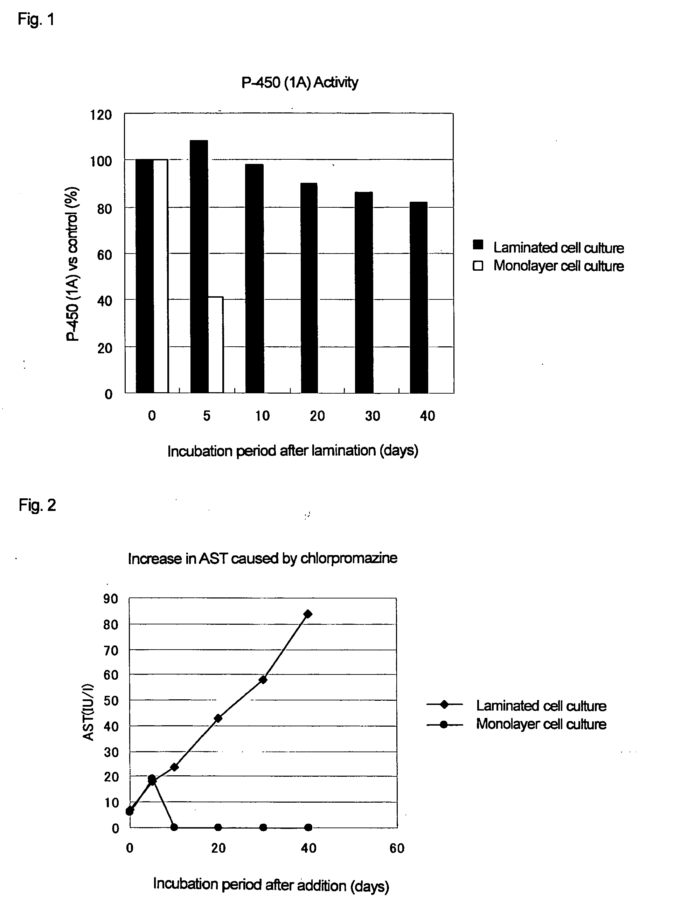

The P-450 activity (P-450(1A)) in the laminated cell culture obtained in Example 2 and the hepatocytes cultured as monolayer culture was measured over time according to the method described in "Lecture of Biochemical Experiments", Vol. 2, Asakura Publishing, p.453. The results are shown in Fig. 1.

As shown by the results indicated in Fig. 1, the rat primary hepatocytes contained in the laminated cell culture maintained the P-450 activity for 40 days. In contrast, in the primary hepatocytes cultured as monolayer culture (only the rat primary hepatocytes were inoculated), the P-450 activity was no longer observed after 5 days.

### [Example 4] Analysis of hepatic metabolism product using laminated cell culture

The laminated cell culture prepared in Example 2 and the hepatocytes cultured as monolayer culture was added with 0.1 µg/ml of chloropramazine, and the amount of AST released into the medium was measured over time. The results are shown in Fig. 2. As shown by the results indicated in Fig. 2, AST was not detected in the primary hepatocytes cultured as monolayer culture as in Example 4 and the cells became extinct on the 5th day, whilst an increase in the amount of AST was observed in the laminated cell culture according to the present invention.

### [Example 5] Preparation of laminated cell culture system

40,000 of estrogen-like substance detection cells (MCF-7) were inoculated on a 12-well microplate and cultured for 7 days under the conditions of 37°C and 5% CO₂ to prepare an MCF-7 cell layer.

The carrier for cell culture prepared in Example 1 was adhered to bottom of a 20-mm petri dish (FALCON, No. 2163) with a small amount of sterilized water, and 40,000 cells of rat primary hepatocytes (collected by the method described in "Techniques of Tissue Culture, Second edition", Ed. by Japan Tissue Culture Association, Asakura Publishing, p.135) were inoculated on the carrier for cell culture and cultured for 24 hours under the conditions of 37°C and 5% CO₂. In the same manner, 20,000 cells of rat vascular endothelial cells A-10 (Dainippon Pharmaceutical Co., Ltd.) were inoculated on the carrier for cell culture prepared in Example 1 and cultured for 72 hours under the conditions of 37°C and 5% CO₂.

The rat primary hepatocyte cell culture obtained was carefully placed on the MCF-7 cell layer on the 12-well microplate so that the side of the growth of the rat primary hepatocyte was on the side of the MCF-7 cells, and cultured for 24 hours under the conditions of 37°C and 5% CO₂. After the culture, 1 mM ethylenediaminetetrasulfonic acid was added to the culture, the cells were cultured for 10 minutes under the conditions of 37°C and 5% CO₂, and the chitosan membrane coming up to the upper surface was removed by using a pair of tweezers. On the resulting cell culture, the cell culture containing the rat vascular endothelial cell layer was placed with the cell growing side down, and the cells were similarly cultured for 24 hours under the conditions of 37°C and 5% CO₂. The culture was further added with 1 mM ethylenediaminetetrasulfonic acid, the cells were cultured for 10 minutes under the conditions of 37°C and 5% CO₂, and the chitosan membrane coming up to the upper surface was removed by using a pair of tweezers to obtain a laminated cell culture system.

The laminated cell culture of rat primary hepatocytes and rat vascular endothelial cells may also be prepared on a cell culture insert (for 12-well plate, BD FALCON, No. 354490). For the above culture, the cell culture insert is used by being inserted in each well of a microplate in which MCF-7 cells are cultured.

### [Example 6] Detection of endocrine-like substance

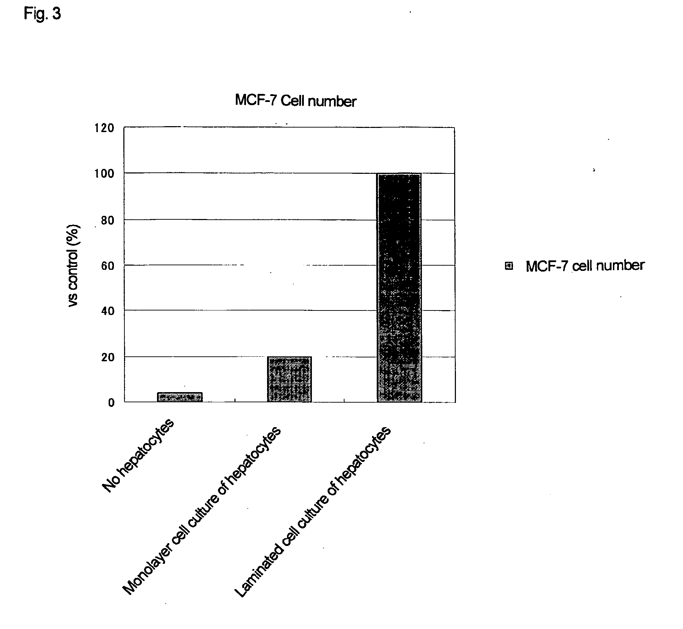

A medium (E-MEM) was added to the laminated cell culture system obtained in Example 5, 2,4-diisopropylnaphthalene (substance positive in the rat uterus proliferation test) was further added to the medium in an amount of 0.1 *µ*g/ml, and the cells were cultured for 14 days under the conditions of 37°C and 5% CO₂. As control cultures, culture with no hepatocytes (only the MCF-7 cell layer) and culture comprising monolayer culture of the rat primary hepatocytes laminated with the MCF-7 cell layer on the cell insert were similarly cultured. Each number of cells after the culture is shown in Fig. 3. Each number of cells shown in Fig. 3 is represented by a percentage value on the basis of the number of MCF-7 cells cultured as laminated culture, which is taken as 100. When 2,4-dinitronaphthalene produced by long-term metabolism of 2,4-diisopropylnaphthalene acts stronger, the number of cells increases. As shown by the results indicated in Fig. 3, in the laminated cell culture system according to the present invention, the number of MCF-7 cells gave the maximum value.

## Claims

1. A laminated cell culture obtained by lamination of two or more cell layers, wherein at least one of the two or more cell layers is a cell layer containing hepatocytes.

2. The laminated cell culture according to claim 1, wherein the at least one of the two or more cell layers is a cell layer containing one or more kinds of cells selected from the group consisting of vascular endothelial cells, vascular smooth muscle cells, adipocytes, hemocytes, and macrophages.

3. The laminated cell culture according to claim 2, which is obtained by adjacently laminating the cell layer containing hepatocytes and the cell layer containing one or more kinds of cells selected from the group consisting of vascular endothelial cells, vascular smooth muscle cells, adipocytes, hemocytes, and macrophages.

4. The laminated cell culture according to any one of claims 1 to 3, wherein at least one of the two or more cell layers is a cultured cell layer which is cultured by using a carrier for cell culture having a multi-layer structure adjacently comprising a water-containing polymer gel layer and a gel layer containing an anionic polysaccharide and a polyvalent metal ion.

5. The laminated cell culture according to claim 4, wherein the lamination of the cell layers comprises the step of solubilizing the cell culture containing the carrier for cell culture having a multi-layer structure adjacently comprising a water-containing polymer gel layer and a gel layer containing an anionic polysaccharide and a polyvalent metal ion.

6. A method for safety evaluation of a substance, which uses the laminated cell culture according to any one of claims 1 to 5.

7. A method for safety evaluation of a substance, which comprises:
(1) the step of adding the substance to a medium containing the laminated cell culture according to any one of claims 1 to 5, and
(2) the step of analyzing a product in the medium after the addition of the substance.

8. A kit for safety evaluation of a substance, which comprises the laminated cell culture according to any one of claims 1 to 5.

9. A cell culture system comprising a cell population for metabolism activation and a cell population containing cells for detection of an endocrine-like substance.

10. A laminated cell culture system obtained by lamination of:
(a) a laminated cell culture obtainable by lamination of two or more cell layers; and (b) a cell layer containing cells for detection of an endocrine-like substance.

11. The laminated cell culture system according to claim 10, wherein at least one of the two or more cell layers of the laminated cell culture is a cell layer containing hepatocytes, and at least one of the other layers is a cell layer containing one or more kinds of cells selected from the group consisting of vascular endothelial cells, vascular smooth muscle cells, adipocytes, hemocytes, and macrophages.

12. The laminated cell culture system according to claim 11, wherein the lamination of the two or more cell layers is adjacently laminating the cell layer containing hepatocytes and a cell layer containing one or more kinds of cells selected from the group consisting of vascular endothelial cells, vascular smooth muscle cells, adipocytes, hemocytes, and macrophages.

13. The laminated cell culture system according to any one of claims 10 to 12, wherein each of at least one of the two or more cell layers and the cell layer containing cells for detection of an endocrine-like substance is a cultured cell layer which is cultured by using a carrier for cell culture having a multi-layer structure adjacently comprising a water-containing polymer gel layer and a gel layer containing an anionic polysaccharide and a polyvalent metal ion.

14. The laminated cell culture system according to claim 13, wherein at least one step of the lamination of the cell layers comprises solubilization of the cell culture containing the carrier for cell culture having a multi-layer structure adjacently comprising a water-containing polymer gel layer and a gel layer containing an anionic polysaccharide and a polyvalent metal ion.

15. A method for detecting an endocrine-like substance, which uses the cell culture system according to any one of claims 9 to 14.

16. A method for detecting an endocrine-like substance, which comprises:
(1) the step of adding a test substance to a medium containing the laminated cell culture system according to any one of claims 9 to 15, and
(2) the step of analyzing an endocrine-like action generated in the laminated cell culture system after the addition of the test substance.

17. A kit for detecting an endocrine-like substance, which comprises the laminated cell culture system according to any one of claims 9 to 15.
